(19) Europäisches Patentamt
European Patent Office
Office européen des brevets

(11) Publication number: **0 124 200 B1**

# (12) EUROPEAN PATENT SPECIFICATION

(45) Date of publication of patent specification: **13.06.90**

(51) Int. Cl.$^5$: **A 61 L 33/00,** C 08 J 7/04

(21) Application number: **84300805.3**

(22) Date of filing: **08.02.84**

## (54) Heparinization of plasma treated substrates.

(30) Priority: **27.04.83 US 488911**

(43) Date of publication of application:
**07.11.84 Bulletin 84/45**

(45) Publication of the grant of the patent:
**13.06.90 Bulletin 90/24**

(84) Designated Contracting States:
**BE DE FR GB IT NL SE**

(56) References cited:
**EP-A-0 051 354**
**GB-A-1 357 011**
**US-A-3 639 123**
**US-A-3 810 781**

(73) Proprietor: **Becton, Dickinson and Company**
**Mack Centre Drive P.O. Box 2224**
**Paramus New Jersey 07652-1149 (US)**

(72) Inventor: **Williams, Joel L.**
**1306 Walnut Street**
**Cary North Carolina (US)**
Inventor: **Dunn, Terry S.**
**1300 Canterbury Road**
**Raleigh North Carolina (US)**
Inventor: **O'Connell, James P.**
**1909 White Plains Road**
**Chapel Hill North Carolina (US)**
Inventor: **Montgomery, David**
**801 Brookgreen Drive**
**Cary North Carolina (US)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 124 200 B1

**Description**

The present invention relates generally to methods of reducing thrombogenicity associated with polymer resin articles. More particularly, the present invention relates to a method for producing a heparin coated polymeric article wherein the polymeric article is treated with a plasma prior to attachment of the heparin.

US Patent No. 3,846,353, to Grotta indicates that polymers, both natural and synthetic and particularly certain synthetic plastics have come to the fore as preferred materials for prosthetic devices. Their major drawback, however, is their thrombogenicity. Even though plastics are used in various apparatus such as heart-lung machines, kidney machines, and artificial heart valves and patches, the tendency of these materials to cause coagulation necessitates the use of anticoagulants such as heparin. Even such plastics as Teflon (trade mark for polytetrafluoroethylene) and the silicone rubbers which are more compatible with blood than most plastics, still show thrombogenic characteristics.

The first real advance in the preparation of nonthrombogenic materials was described by Dr. Vincent Gott. The method used by Dr. Gott comprised treating a graphited surface first with Zephiran (trade mark for benzalkonium chloride) and then with heparin. Materials treated in this way were nonthrombogenic *in vivo* for long periods of time.

The major disadvantage, however, with these materials, was that the method could only be practiced on rigid plastics. Thus a need still exists for a suitable flexible nonthrombogenic plastics material, as well as a method for producing it.

Various methods for introducing antithrombogenicity involve chemically bonding a quaternary ammonium salt to a polymeric surface. Illustrative procedures are given in US Patent No. 3,634,123 to Eriksson wherein an article having a plastics surface is heated to near or above its softening point in an aqueous solution of a cationic surface active agent such as a long chain alkylamine hydrohalide. In such manner, the surface active agent permeates and thereby becomes affixed to the resin surface. In addition, the hydrocarbon portion of the surface-active agent is thought to become bound to the surface of the plastics. Subsequent digestion of the plastics articles with an aqueous solution of heparin provides articles of enhanced antithrombogenicity.

A further improvement is described in US Patent No. 3,810,781 to Eriksson, wherein heparinized plastics surfaces are stabilized by cross-linking the bonded heparin molecules with dialdehydes. Some improvement in stability results, but not all of the bonded heparin is impervious to desorption by washing.

The binding of substantially greater quantities of heparin to the polymer surfaces is described in US Patent No. 4,349,467 to Dudley, wherein the step of heparinzation is carried out with aqueous solutions of heparin of 5% or greater concentration. Using this procedure, up to 18 microgram/cm of heparin is bound to a polyurethane surface through the surface active agent.

Many of the drawbacks and disadvantages of earlier methods for rendering polymeric surfaces nonthrombogenic are eliminated by the teaching of the above-named patents.

There remains, however, a need for methods to adhere higher quantities of heparin, impervious to desorption, to polymeric surfaces for use in articles which will be in contact with blood for prolonged periods of time.

These needs are met by the method of the current invention, which includes surface activation of a polymeric substrate using plasma generated by radio frequency discharge.

The process of formation of a plasma by electromagnetic activation of a gas by either a glow discharge or a corona discharge, and the use of such plasma for activating polymeric surfaces is known to accomplish various purposes. For example, US Patent No. 3,663,265 to Lee teaches deposition of vaporized polymeric material onto substrate surfaces by treatment of the polymeric material with a plasma from an inert gas whereby the polymer is vaporized and contacted with the substrate to form the coating. In US Patent No. 4,091,166 to Kubacki, a plastics surface is treated with a plasma of boron trifluoride, optionally admixed with an organic monomer to deposit a boron trifluoride containing coating. US Patent No. 3,415,683 to Coffman discloses formation of deposits from organic materials on substrates such as bar metals in a corona reaction. US Patent No. 3,518,108 to Heiss teaches formation of polymer coatings from aromatics, aliphatics and silicones in a plasma of inert gas or hydrogen.

US Patent No. 3,776,762 to Bernath teaches glow discharge deposition of fluorocarbon polymeric coatings onto metallic or non-metallic surfaces by passing DC current through a low pressure atmosphere of the monomer. US Patent No. 4,326,532 to Hammar discloses the use of a plasma for priming the surface of a polymeric resin for deposition of a coating of chitosan receptive to subsequent heparin binding.

The invention comprises a method to improve adherence of heparin to a polymeric surface. The polymeric article heparinized according to the present invention typically has improved compatibility with blood due to the increased level of heparin that can be adhered to the surface. The improvement results from a plasma treatment of the plastics surface before treatment with a cationic surface active agent.

The present invention provides a method of preparing a polymer article with reduced thrombogenicity, which method comprises the steps of surface activation of a polymeric substrate using plasma generated by ionization of a gas by radio frequency discharge, treatment of the surface-activated polymeric substrate with a surface active agent having a cation of the general formula $R^1R^2R^3R^4N^+ \cdot X^-$ (wherein $R^1$ is $C_{12}$—$C_{18}$ alkyl, $R^2$, $R^3$ and $R^4$ are each independently hydrogen or $C_1$—$C_6$ alkyl, and $X^-$ is a negative monovalent ion)

for affixing the surface active agent to the activated surface by absorption of the surface active agent at the surface of the plasma-treated polymeric substrate, and then heparinization of the surface active agent-treated surface of the polymeric substrate.

The plasma can be generated by ionization of a gas at low pressure by a radio frequency discharge. The frequency and power used to generate the plasma and the time of plasma treatment are variable over wide ranges. The plasma-treated polymeric surface thus obtained can then be subjected to heparinization according to known methods, whereby up to 10-fold more heparin is bound to the polymeric surface than occurs with untreated polymers. In addition, the heparin is usually bound more firmly, affording highly durable heparin surfaces which are resistant to removal of the heparin by such operations as washing or rubbing. Another advantage of the present invention is that normally no effect on the mechanical or penetration characteristics of prosthetic devices such as catheters is seen. Furthermore, in contrast to prior art methodology, the process of the present invention is relatively inexpensive and clean.

The polymeric resin materials which serve as the substrate to be treated by the method of this invention may be a polymeric resin, natural or synthetic, conventionally used to fabricate articles commonly used in contact with blood. For example, catheters, artificial blood vessels, valves and like prosthetics are frequency fabricated from a polyethylene, polacrylic, polypropylene, polyvinyl chloride, polyamide, polyurethane, polyvinylpyrodidone, polyvinyl alcohol, cellulose acetate, polystyrene, polytetrafluorethylene, polyester such as polyethylene terephthalate, silicone rubber, natural rubber, polycarbonate and like polymeric resins and hydrogels, thereof. The resin substrate may be rigid or flexible in character, cellular or non-cellular, porous or non-porous. Also within the scope of the invention is the treatment of a coating of such a polymer resin on a metal or ceramic material.

The polymeric resin substrate may be first formed into any desired shape, size or configuration. Representative forms include valves, pins, containers, sleeves, connectors, medical-surgical tubing, prosthetic devices and the like of any size. Alternatively, the polymeric resin may be first treated by the method of this invention and subsequently fabricated into the desired form.

In accordance with the method of this invention, the polymeric resin substrate is subjected to a plasma treatment before heparinization. This plasma treatment may be carried out in a plasma generator, as, for example, those described in US Patent No. 3,847,652. The plasma may be generated from a variety of gases or mixtures thereof. Gases frequently used include hydrogen, helium, ammonia, nitrogen, oxygen, neon, argon, krypton and xenon. Gas pressures are advantageously maintained at 5 mm of Hg or below, preferably from about 0.1 to about 1.0 mm of Hg, more preferably 10 to 300 micron of Hg, in order to benefit from reduced voltage requirements.

A wide range of power settings, radio frequencies and durations of exposure of the polymeric surface to the plasma may be used. Ranges for these three parameters which provide advantageous results are DC or AC power levels from 10 up to about 200 watts, from about 1 to about 50 megahertz and from about 0.1 to about 30 minutes, respectively. Preferred ranges are 10—50 watts, 10—20 megahertz and 2—10 minutes respectively.

The plasma-treated polymer is then treated with a surface active agent before heparinization. To the plasma surface thus activated by the plasma treatment there is affixed by absorption on the surface a quanternary ammonium salt of the general formula (I),

$$R^1R^2R^3R^4N^+ \cdot X^-,$$

wherein $R^1$ is alkyl of 12—18 carbon atoms, $R^2$, $R^3$ and $R^4$ are each independently selected from hydrogen atoms and lower alkyl groups of 1 to 6 carbon atoms, and X is a negative monovalent ion, such as halogen. In preferred embodiments of this invention, the group $R^2$ is lower alkyl and $R^3$ and $R^4$ are hydrogen. In a particularly preferred embodiment, $R^2$ is methyl, $R^3$ and $R^4$ are hydrogen and X is chlorine.

Specific examples for the surface active agent include dodecylmethylammonium chloride, tetradecylmethylammonium chloride, hexadecylmethylammonium chloride, octadecylmethylammonium chloride or dodecylhexylammonium chloride, especially dodecylmethylammonium chloride.

The compound of Formula (I) is affixed to the polymeric resin substrate by permeating the compound throughout the molecular structure of the resin substrate, that is by chemisorption. While not willing to be bound by any theory, we believe that the $C_{12}$ to $C_{18}$ alkyl chain portion of the compound of formula (I) may also bind to the polymeric resin substrate which has been chemically altered by the plasma treatment. The compound (I) may be chemisorbed into the polymeric resin substrate by steeping the substrate in a dispersion of the compound (I). In this steeping operation, the concentration of compound (I) is not critical, but is advantageously maintained within the range of 0.01% to 2.0% by weight. Steeping may be suitably carried out for 1 to 24 hours and at ambient or at elevated temperatures up to or slightly above the softening point temperature for the resin substrate, the softening point temperature being the temperature at which the surface of the resin substrate becomes pliable due to the additional mobility of the substrate molecules. Following fixation of the quaternary ammonium salt compound (I) to the surface of the polymeric resin substrate, excess compound (I) may be removed by washing with distilled water or saline solutions.

After the fixation, the polymeric resin substrate is preferably heparinized by immersion in an aqueous solution of sodium heparin. The temperature at which immersion is advantageously carried out is within

the range of from about room temperature to about 80°C, but less than the softening point temperature for the resin substrate. The length of immersion is dependent on the temperature used, and might be from 1 to 24 hours, but is generally long enough to permit the substrate to pick up at least about 0.1 International Unit of heparin per square centimeter of substrate surface. At a temperature of about 70°C, for example, this is usually accomplished in about 1 hour, using a heparin solution with a concentration of from about 1% to about 15%, preferably from about 8 to about 10%, of sodium heparin (all percentages herein are by weight unless otherwise indicated).

During the preferred heparinization, the negative ion of the sodium heparin complexes with the positive ion of the compound I according to the scheme:

$$R^1R^2R^3R^4N^+ \cdot X^- + Na^+A^- = R^1R^2R^3R^4N^+ \cdot A^- + Na^+X^-$$

wherein A represents the active heparin moiety, that is, the negative ion of a salt of heparin, and $R_1$, $R_2$, $R_3$, $R_4$ and X are as previously defined.

Following the heparinization step, the polymeric resin can be removed from the heparin and rinsed thoroughly with distilled water.

The heparinized surface of the polymeric resin can be stabilized toward desorption in the presence of blood by treatment with a dialdehyde to cross-link functional groups of heparin. This cross-linking of functional groups in different heparin units is suitably accomplished when the heparinized surface is digested with an aqueous solution of a dialdehyde over a concentration range of 0.1% to 5.0%. The aldehyde is preferably one with 2 to 6 carbon atoms, for instance glutaraldehyde. It is most advantageous to maintain contact between the heparinized surface and the dialdehyde solution for a time period of about 1 to about 6 hours at a temperature of ambient to about 80°C. The heparinized surface thus stabilized is preferably removed from the bath, washed thoroughly with distilled water and dried before being brought into contact with blood.

In a preferred embodiment of the present invention, a polytetrafluoroethylene or polyethylene substrate is maintained in an oxygen plasma generated at about 13.56 MHz and about 50 watts for about 10 minutes. The activated substrate is then steeped for about 16 hours at about 65°C in an aqueous approximately 15% solution of dodecylmethyl ammonium chloride. After washing with distilled water, the substrate with its affixed quaternary ammonium salt is treated at about 65°C for about 16 hours with an approximately 9% solution of sodium heparin. After washing with distilled water, the stabilization step is carried out with an approximately 1% aqueous solution of glutaraldehyde at about 60°C for about 2 hours.

The amount of heparin bound to the surface of the polymeric resin substrate can be determined by a standard procedure based on the quantitative removal of the dye Azure A by the reactive sites of the bound heparin. An example of a suitable methodology is as follows. Heparinized polymeric substrate of known surface area (between 2 and 35 $cm^2$) is exposed to 5 ml of a 0.001% aqueous solution of Azure A for 45 minutes at 25°C. The quantity of dye removed from the solution is determined by spectrophotometric readings made at 630 nm, with a light path of 1 cm. The dye removed is converted to equivalent amounts of heparin by means of a standard curve prepared by reacting graded amounts of heparin (1—100 micrograms) with five ml of 0.001% Azure A in water, removing the insoluble heparin dye complex by extraction with 4 ml of cyclohexane, and quantifying the amounts of dye removed spectrophotometrically. The standard curve is then prepared by plotting amount of heparin added versus absorbance at 630 nm. The amount of heparin present on the tubing can then be determined by dividing the amount of heparin removed (derived from the standard curve) by the total surface area of the sample.

Polytetrafluoroethylene articles heparinized according to the method of the present invention were tested for blood compatibility by the procedure described in our US Patent 4,367,749, and found to be very satisfactory.

The following examples are provided to illustrate further the advantages of this invention, but the conditions and materials used and the amounts thereof are not limiting of the scope of the invention.

Example 1

One hundred 1.25 inch (say 3.2 cm) radiopaque polytetrafluoroethylene catheters each weighing 20 g were placed in a plasma generator. The system was evacuated for 6 minutes to a pressure of 120 micron of Hg, then an oxygen bleed was started and maintained for 1 minute at a pressure of 180 micron of Hg. A plasma was initiated and maintained at 13.56 MHz and 50 watts power for 10 minutes. The chamber was air quenched, opened, and the catheters heparinized according to the following sequential three-step procedure.

1. Steeping in a 15% aqueous solution of dodecamethylmethyl ammonium chloride, pH 7.5, 16 hours, 65°C, followed by thorough rinsing in distilled water and drying with nitrogen.

2. Digestion in a 9% aqueous solution of sodium heparin for 16 hours at 65°C, followed by thorough rinsing with distilled water and drying with nitrogen.

3. Submersion in a 1% aqueous solution of glutaraldehyde for 2 hours at 60°C, followed by thorough rinsing with distilled water, 5% aqueous Triton x 405, and distilled water. The catheters were dried with nitrogen and stored in a vacuum oven at 25°C.

Example 2

A polystyrene microtiter was placed in a plasma generator and the system evacuated to 20 micron of Hg. Ammonia was bled in at such a rate that a pressure of 200 micron of Hg was established and maintained. After a 5 minute flush at this pressure, a plasma was initiated at a frequency of 20 MHz and 40 watts power. The plasma was maintained for 5 minutes. The radio frequency was turned off and, after an additional 5 minutes flow of ammonia, the system was opened, the sample removed and heparinized as described in Example 1.

Example 3

Twelve polytetrafluoroethylene catheters were placed in the plasma generator. The system was evacuated, and an oxygen bleed of 20 ml per minute giving a pressure of 180 micron of Hg was maintained for 2 minutes. A plasma was generated and maintained for 10 minutes at 13.56 MHz and 50 watts power. The system was quenched with air and the catheters removed and heparinized as in Example 1.

Example 4

Polytetrafluoroethylene tubing, 22 gauge, was cut into 3 foot (say 76 cm) lengths and placed in a glow discharge chamber. The system was evacuated for 5 minutes and flushed with oxygen at 1 mm of Hg for 30 seconds. A flow of oxygen to produce a pressure of 180 micron of Hg was initiated, and plasma was generated for 10 minutes at 50 watts and 13.56 MHz. The system was quenched with air, opened, the substrate repositioned in the chamber to ensure total exposure to the plasma, and the plasma generated a second time in the same way. The tubing was removed from the chamber and heparinized as in Example 1.

Example 5

Into a plasma generator were placed the following:

| | |
|---|---|
| 8 | 20 g, 2.5 inch (say 6.4 cm) radiopaque polytetrafluoroethylene catheters (non siliconized) |
| 4 | 16 g, clear polytetrafluoroethylene catheters |
| 10 | 16 g polytetrafluoroethylene guidewires |
| 10 | pieces of polytetrafluoroethylene tubing. |

The chamber was evacuated and an oxygen bleed giving a pressure of 180 micron of Hg was maintained for 2 minutes. A plasma was then initiated using 50 watts of power and a frequency of 13.56 MHz maintained for 10 minutes. These materials were heparinized according to the procedure of Example 1. When these materials were exposed to a 0.001% aqueous solution of Azure A, heavy staining occurred, indicating high surface concentrations of heparin.

Example 6

Into a plasma generator were placed a group of 2, 2.5 and 4 inch (say 5.1, 6.4 and 10.2 cm) radiopaque polytetrafluoroethylene catheters and 5 polytetrafluoroethylene coated guide wires. These were subjected to an oxygen plasma according to the procedure described in Example 5, and the plasma treated materials were heparinized according to the procedure of Example 1. The heparinized surfaces were subjected to an amount of rubbing equivalent to that which occurs in a typical human insertion. By comparison with samples which were not rubbed, it is seen, based on the Azure A staining procedure, that the heparinized surface retains most of the heparin after rubbing. This indicates a very durable heparin surface.

Example 7

This experiment was run in duplicate, giving the results shown in the following chart, runs 1 and 2.

Four 4-foot (say 102 cm) samples each of radiopaque polytetrafluoroethylene (ptfe) tubing and clear polyethylene tubing were subjected to an oxygen plasma generated at 50 watts of power and a frequency of 13.56 MHz. Treatment durations of 1 and 10 minutes and oxygen pressures of 170 micron of Hg and 500 micron of Hg were used. The samples were heparinized according to the procedure of Example 1, except glutaraldehyde cross linking was not done. The tubing lumens were washed with hot 6 m sodium chloride solution to remove all heparin thereon, since it is desired to quantitate only the heparin on the outer surface of the tubings (which were exposed to plasma). The following chart summarizes the quantities of heparin bound to the surfaces of the tubing.

| Substrate | Oxygen plasma treatment | | Bound heparin microg./cm$^2$ | | |
|---|---|---|---|---|---|
| | Time min. | Pressure micron Hg | Run 1 | Run 2 | Control* |
| polyethylene | 1 | 170 | 5.70 | 6.77 | 7.37 |
| polyethylene | 10 | 170 | 5.74 | 6.82 | |
| polyethylene | 1 | 500 | 8.71 | 10.30 | |
| polyethylene | 10 | 500 | 7.94 | 9.39 | |
| ptfe | 1 | 170 | 1.24 | 1.50 | 0.22 |
| ptfe | 10 | 170 | 2.31 | 2.76 | |
| ptfe | 1 | 500 | 1.66 | 2.01 | |
| ptfe | 10 | 500 | 4.63 | 5.51 | |

*heparin bound to substrate not subjected to oxygen plasma.

Example 8

Polytetrafluoroethylene tubing of .02 mm inside diameter was placed in a plasma generator and the system was evacuated. Nitrogen was bled in at such a rate that a pressure of 180 micron of Hg was established and maintained. After a 2 minute flush at this pressure, a plasma was generated and maintained for 10 minutes at 40 watts of power and a frequency of 13.56 MHz. The radio frequency was turned off and, after an additional 10 minutes flow of nitrogen, the system was opened, the samples removed and heparinized as described in Example 1. The articles thus treated with plasma followed by heparinization were compared for blood compatibility with untreated controls and with controls heparinized without plasma treatment. The results of these comparative experiments are shown in Chart II.

Chart II

| Untreated teflon controls | Heparinized (no plasma pretreatment) |
|---|---|
| 9.4 minutes | 18 minutes |
| 13.2 | 22 |
| 13.8 | 26 |
| 15.2 | 29 |
| 15.7 | 59 |
| Average=13.5 | Average=30.8 minutes |

| Heparinized (with plasma pretreatment) |
|---|
| 47 minutes |
| 92 |
| 120+ (truncated) |
| 136 |
| 139 |
| Average=106.8 minutes |

Thus an average 8 fold increase in blood compatibility over untreated controls and a 3—4 fold increase over heparinized articles not pretreated with plasma were achieved.

**Claims**

1. A method of preparing a polymer article with reduced thrombogenicity, which method comprises the steps of surface activation of a polymeric substrate using plasma generated by ionization of a gas by radio frequency discharge, treatment of the surface-activated polymeric substrate with a surface active agent having a cation of the general formula $R^1R^2R^3R^4N^+ \cdot X^-$ (wherein $R^1$ is $C_{12}$—$C_{18}$ alkyl, $R^2$, $R^3$ and $R^4$ are each independently hydrogen or $C_1$—$C_6$ alkyl, and $X^-$ is a negative monovalent ion) for affixing the surface active agent to the activated surface by absorption of the surface active agent at the surface of the plasma-treated polymeric substrate, and then heparinization of the surface active agent-treated surface of the polymeric substrate.

2. A method according to Claim 1 in which the polymeric substrate comprises silicone, polyethylene, polypropylene, polyurethane, polyacrylic, polyamide, polyester, polyvinyl pyrrolidone, polyvinyl alcohol, cellulose acetate, polyvinyl chloride or polytetrafluoroethylene, preferably polytetrafluoroethylene or polyethylene.

3. A method according to Claim 1 or 2 wherein the plasma is generated from a gas which comprises oxygen, ammonia, hydrogen, helium, neon, argon, krypton, xenon, nitrogen or mixtures thereof.

4. A method according to Claim 3 wherein the gas is maintained at a chamber pressure of from 10 to 300 micron of mercury during generation of the plasma.

5. A method according to any preceding Claim wherein the plasma is generated using a radio frequency discharge, preferably from 1 to 50 megahertz.

6. A method according to Claim 5 wherein the plasma is generated with a power of from 10 to 200 watts.

7. A method according to any preceding Claim wherein the polymeric substrate is subjected to the plasma for a period of from for 0.1 to 30 minutes.

8. A method according to Claim 1 wherein a polytetrafluoroethylene substrate is subjected to an oxygen plasma generated by a discharge of about 50 watts at a radio frequency of about 13.56 megahertz and maintained for about 10 minutes with the oxygen at a pressure of about 180 micron of Hg.

9. A method according to any preceding claim wherein after heparinization, the substrate is treated with an aqueous solution of a dialdehyde.

10. A method according to any preceding Claim, wherein $R^2$ is lower alkyl of 1 to 6 carbon atoms, $R^3$ and $R^4$ are each hydrogen and $X^-$ is halide, preferably chloride.

11. A method according to Claim 10 wherein the surface active agent comprises dodecylmethylammonium chloride, tetradecylmethylammonium chloride, hexadecylmethylammonium chloride, octadecylmethylammonium chloride or dodecylhexylammonium chloride, preferably dodecylmethylammonium chloride.

12. A method according to any preceding Claim, wherein the aqueous concentration of the cationic surface active agent is from 0.01 to 2.0%.

13. A method according to any preceding Claim, wherein the treatment with the surface active agent is carried out by steeping of the substrate in an aqueous dispersion of the surface active agent is carried out for 1 to 24 hours and/or the steeping is carried out at ambient temperature or at an elevated temperature up to or slightly above the softening temperature of the substrate.

14. A method according to any preceding Claim, wherein for the heparinization the substrate is immersed in aqueous solution of from 1 to 15 per cent of sodium heparin.

15. A method according to any preceding claim, wherein the treatment with the surface active agent is carried out by immersion at a temperature from ambient temperature to 80°C and/or for 1 hour to 24 hours.

16. A method according to claim 9 wherein the dialdehyde contains from 2 to 6 carbon atoms and is preferably glutaraldehyde.

17. A method according to claim 9 or 16, wherein the dialdehyde is used in an aqueous solution of from 0.01 to 5% and/or the temperature of the dialdehyde aqueous solution is from ambient temperature to 80°C.

18. A method according to claim 9, 16 or 17, wherein the substrate is contacted with the dialdehyde aqueous solution for from 1 to 6 hours.

19. A method according to claim 1, the method comprising the sequential steps of:

(1) subjecting a polytetrafluoroethylene substrate to an oxygen plasma wherein the plasma is generated by a discharge of about 50 watts at a radio frequency of about 13.56 megahertz, the discharge being maintained for about 10 minutes with the oxygen at a pressure of about 180 micron of Hg;

(2) steeping the substrate in an aqueous solution of dodecylmethyl ammonium chloride of about 15% concentration for about 16 hours at about 65°C;

(3) washing the substrate thoroughly in distilled water;

(4) immersing the substrate in an aqueous solution of sodium heparin of about 9% concentration for about 16 hours at about 65°C;

(5) washing the substrate thoroughly in distilled water;

(6) digesting the substrate in an aqueous solution of glutaraldehyde of about 1% concentration for about 2 hours at about 60°C;

(7) washing the substrate in distilled water.

**Patentansprüche**

1. Verfahren zur Herstellung eines Polymer-Artikels mit verminderter Thrombogenizität, das folgende Schritte umfaßt: Oberflächenaktivierung eines polymeren Substrats unter Verwendung eines durch Ionisieren eines Gases durch Hochfrequenzentladung erzeugten Plasmas, Behandlung des oberflächenaktivierten polymeren Substrats mit einem oberflächenaktiven Mittel mit einem Cation der allgemeinen Formel $R^1R^2R^3R^4N^+ \cdot X^-$ (in der $R^1$ $C_{12}$—$C_{18}$-Alkyl, $R^2$, $R^3$ und $R^4$ jeweils uabhängig Wasserstoff oder $C_1$—$C_6$-Alkyl und $X^-$ ein negatives einwertiges Ion ist) zum Befestigen des oberflächenaktiven Mittels an die aktivierte Oberfläche durch Absorption des oberflächenaktiven Mittels an der Oberfläche des plasmabehandelten polymeren Substrats, sodann Heparinisation der mit dem oberflächenaktiven Mittel behandelten Oberfläche des polymeren Substrats.

2. Verfahren nach Anspruch 1, dadurch gekennzeichnet, daß das polymere Substrat Silicon, Polyethylen, Polypropylen, Polyurethan, Polyacryl, Polyamid, Polyester, Polyvinylpyrrolidon,

Polyvinylalkohol, Celluloseacetat, Polyvinylchlorid oder Polytetrafluorethylen, vorzugsweise Polytetrafluorethylen oder Polyethylen umfaßt.

3. Verfahren nach Anspruch 1 oder 2, dadurch gekennzeichnet, daß das Plasma aus einem Gas erzeugt wird, das Sauerstoff, Ammoniak, Wasserstoff, Helium, Neon, Argon, Krypton, Xenon, Stickstoff oder Mischungen daraus umfaßt.

4. Verfahren nach Anspruch 3, dadurch gekennzeichnet, daß das Gas während der Erzeugung des Plasmas auf einem Kammerdruck von 10—300 µm Hg gehalten wird.

5. Verfahren nach einem der vorherigen Ansprüche, dadurch gekennzeichnet, daß das Plasma unter Verwendung einer Hochfrequenzentladung erzeugt wird, vorzugsweise von 1—50 MHz.

6. Verfahren nach Anspruch 5, dadurch gekennzeichnet, daß das Plasma mit einem Strom von 10—200 Watt erzeugt wird.

7. Verfahren nach einem der vorherigen Ansprüche, dadurch gekennzeichnet, daß das polymere Substrat dem Plasma über eine Zeit von 0,1 bis 30 Minuten ausgesetzt wird.

8. Verfahren nach Anspruch 1, dadurch gekennzeichnet, daß ein Polytetrafluorethylensubstrat einem durch eine Entladung von ca. 50 Watt bei einer Hochfrequenz von ca. 13,56 MHz erzeugten Sauerstoffplasma ausgesetzt und etwa 10 Minuten lang mit dem Sauerstoff auf einem Druck von etwa 180 µm Hg gehalten wird.

9. Verfahren nach einem der vorherigen Ansprüche, dadurch gekennzeichnet, daß nach der Heparinisation das Substrat mit einer wässerigen Lösung eines Dialdehyds behandelt wird.

10. Verfahren nach einem der vorherigen Ansprüche, dadurch gekennzeichnet, daß $R^2$ niederes Alkyl von 1—6 Kohlenstoffatomen, $R^3$ und $R^4$ jeweils Wasserstoff und $X^-$ Halid, vorzugsweise Chlorid ist.

11. Verfahren nach Anspruch 10, dadurch gekennzeichnet, daß das oberflächenaktive Mittel Dodecylmethylammoniumchlorid, Tetradecylmethylammoniumchlorid, Hexadecylmethylammoniumchlorid, Oktodecylmethylammoniumchlorid oder Dodecylhexylammoniumchlorid, vorzugsweise Dodecylmethylammoniumchlorid umfaßt.

12. Verfahren nach einem der vorherigen Ansprüche, dadurch gekennzeichnet, daß die wässerige Konzentration des cationischen oberflächenaktiven Mittels zwischen 0,01 und 2,0% beträgt.

13. Verfahren nach einem der vorherigen Ansprüche, dadurch gekennzeichnet, daß die Behandlung mit dem oberflächenaktiven Mittel ausgeführt wird durch 1- bis 24-stündiges Einweichen des Substrats in einer wässerigen Dispersion des oberflächenaktiven Mittels und/oder Einweichen bei Umgebungstemperatur oder einer erhöhten Temperatur bis zur Erweichungstemperatur des Substrats oder geringfügig darüber.

14. Verfahren nach einem der vorherigen Ansprüche, dadurch gekennzeichnet, daß zum Heparinieren das Substrat in eine 1 bis 15% wässerige Natriumheparinlösung getaucht wird.

15. Verfahren nach einem der vorherigen Ansprüche, dadurch gekennzeichnet, daß die Behandlung mit dem oberflächenaktiven Mittel ausgeführt wird bei einer Temperatur zwischen Umgebungstemperatur und 80°C und/oder durch 1- bis 24stündiges Eintauchen.

16. Verfahren nach Anspruch 9, dadurch gekennzeichnet, daß das Dialdehyd zwischen 2 und 6 Kohlenstoffatomen enthält und vorzugsweise Gluraraldehyd ist.

17. Verfahren nach Anspruch 9 oder 16, dadurch gekennzeichnet, daß das Dialdehyd in einer wässerigen Lösung von 0,01 bis 5% benutzt wird und/oder die Temperatur der wässerigen Dialdehydlösung zwischen Umgebungstemperatur und 80°C liegt.

18. Verfahren nach Anspruch 16 oder 17, dadurch gekennzeichnet, daß das Substrat für eine Zeit von 1—6 Stunden mit der wässerigen Dialdehydlösung in Berührung gebracht wird.

19. Verfahren nach Anspruch 1, das die folgenden Schritte umfaßt:

(1) Aussetzen eines Polytetrafluorethylensubstrats der Wirkung eines Sauerstoffplasmas, das dadurch gekennzeichnet, ist, daß es durch eine Entladung von ca. 50 Watt bei einer Hochfrequenz von ca. 13,56 MHz erzeugt wird, wobei die Entladung ca. 10 Minuten lang mit dem Sauerstoff bei einem Druck von etwa 180 µm Hg aufrechterhalten wird;

(2) Einweichen des Substrats in einer wässerigen Dodecylmethylammoniumchloridlösung in einer Konzentration von etwa 15% für etwa 16 Stunden bei ca. 65°C;

(3) Gründliches Waschen des Substrats in destilliertem Wasser;

(4) Eintauchen des Substrats in eine wässerige Natriumheparinlösung in einer Konzentration von ca. 9% für etwa 16 Stunden bei etwa 65°C;

(5) Gründliches Waschen des Substrats in destilliertem Wasser;

(6) Digerieren des Substrats in einer wässerigen Glutaraldehydlösung in einer Konzentration von ca. 1% für eine Dauer von ca. 2 Studen bei etwa 60°C;

(7) Waschen des Substrats in destilliertem Wasser.

## Revendications

1. Procédé de préparation d'un article polymère présentant un caractère thrombogène réduit, lequel procédé comprend les étapes suivantes: activation superficielle d'un substrat polymère, faisant appel à un plasma produit par ionisation d'un gaz par une décharge en radiofréquence, traitement du substrat polymère activé en surface à l'aide d'un agent tensioactif comportant un cation de formule générale

$R^1R^2R^3R^4N^+ \cdot X^-$ (où $R^1$ est un radical alkyle en $C_{12}$—$C_{18}$, les radicaux $R^2$, $R^3$ et $R^4$ représentent chacun, indépendamment les uns des autres, un hydrogène ou un radical alkyle en $C_1$—$C_6$, et $X^-$ est un ion monovalent négatif) pour fixer l'agent tensioactif sur la surface activée, par absorption de l'agent tensioactif sur la surface du substrat polymère traité par le plasma, puis héparinisation de la surface, traitée par l'agent tensioactif, du substrat polymère.

2. Procédé selon la revendication 1, dans lequel le substrat polymère est constitué de silicone, de polyéthylène, de polypropylène, de polyuréthanne, d'un polymère polyacrylique, d'un polyamide, d'un polyester, de polyvinylpyrrolidone, de poly(alcool vinylique), d'acétate de cellulose, de poly(chlorure de vinyle) ou de polytétrafluoréthylène, de préférence de polytétrafluoréthylène ou de polyéthylène.

3. Procédé selon la revendication 1 ou 2, dans lequel le plasma est produit à partir d'un gaz constitué d'oxygène, d'ammoniac, d'hydrogène, d'hélium, de néon, d'argon, de krypton, de xénon, d'azote ou de leurs mélanges.

4. Procédé selon la revendication 3, dans lequel le gaz est maintenu à une pression de chambre comprise entre 1,3 et 40,0 Pa (10 à 300 μm de mercure) pendant la production du plasma.

5. Procédé selon l'une quelconque des revendications précédentes, dans lequel le plasma est produit grâce à l'utilisation d'une décharge en radio fréquence, de préférence de 1 à 50 MHz.

6. Procédé selon la revendication 5, dans lequel le plasma est produit avec une puissance de 10 à 200 W.

7. Procédé selon l'une quelconque des revendications précédentes, dans lequel le substrat polymère est soumis au plasma pendant un laps de temps de 0,1 à 30 minutes.

8. Procédé selon la revendication 1, dans lequel un substrat de polytétrafluoréthylène est soumis à un plasma à l'oxygène produit par une décharge d'environ 50 W, à une radiofréquence d'environ 13,56 MHz, et maintenue pendant environ 10 minutes avec l'oxygène à une pression d'environ 24 Pa (environ 180 μm de mercure).

9. Procédé selon l'une quelconque des revendications précédentes, dans lequel, après héparinisation, le substrat est traité avec une solution aqueuse d'un dialdéhyde.

10. Procédé selon l'une quelconque des revendications précédentes, dans lequel $R^2$ est un radical alkyle inférieur ayant de 1 à 6 atomes de carbone, $R^3$ et $R^4$ représentent chacun un hydrogène, et $X^-$ est un halogénure, de préférence un chlorure.

11. Procédé selon la revendication 10, dans lequel l'agent tensioactif comprend du chlorure de dodécylméthylammonium, du chlorure de tétradécylméthylammonium, du chlorure d'hexadécylméthylammonium, du chlorure d'octadécylméthylammonium ou du chlorure de dodécylhexyl-ammonium, de préférence du chlorure de dodécylméthylammonium.

12. Procédé selon l'une quelconque des revendications précédentes, dans lequel la solution aqueuse de l'agent tensioactif cationique est de 0,01 à 2,0%.

13. Procédé selon l'une quelconque des revendications précédentes, dans lequel le traitement à l'aide de l'agent tensioactif est mis en oeuvre par trempage du substrat dans une dispersion aqueuse de l'agent tensioactif pendant 1 à 24 heures, et/ou le trempage est mis en oeuvre à la température ambiante ou à une température élevée, allant jusqu'à la température de ramollissement du substrat, ou légèrement plus élevée.

14. Procédé selon l'une quelconque des revendications précédentes, dans lequel, pour l'héparinisation, le substrat est immergé dans une solution aqueuse à 1—15% d'héparine sodique.

15. Procédé selon l'une quelconque des revendications précédentes, dans lequel le traitement avec l'agent tensioactif est mis en oeuvre par immersion à une température comprise entre la température ambiante et 80°C, et/ou pendant 1 heure à 24 heures.

16. Procédé selon la revendication 9, dans lequel le dialdéhyde contient de 2 à 6 atomes de carbone et est de préférence le glutaraldéhyde.

17. Procédé selon la revendication 9 ou 16, dans lequel le dialdéhyde est utilisé en solution aqueuse à 0,01—5% et/ou la température de la solution aqueuse du dialdéhyde est comprise entre la température ambiante et 80°C.

18. Procédé selon la revendication 9, 16 ou 17, dans lequel le substrat est mis en contact avec la solution aqueuse du dialdéhyde pendant 1 à 6 heures.

19. Procédé selon la revendication 1, comprenant les étapes successives consistant:

(1) à soumettre un substrat de polytétrafluoréthylène à un plasma à l'oxgène, où le plasma est produit par une décharge d'environ 50 W à une radiofréquence d'environ 13,56 MHz, la décharge étant maintenue pendant environ 10 minutes avec l'oxygène à une pression d'environ 24 Pa (180 μm de mercure);

(2) à tremper le substrat dans une solution aqueuse de chlorure de dodécylméthylammonium à environ 15% de concentration pendant environ 16 heures à environ 65°C;

(3) à bien laver le substrat dans le l'eau distillée;

(4) à immerger le substrat dans une solution aqueuse d'héparine sodique à environ 9% de concentration pendant environ 16 heures à environ 65°C;

(5) à bien laver le substrat dans le l'eau distillée;

(6) à provoquer la digestion du substrat dans une solution aqueuse de glutaraldéhyde à environ 1% de concentration pendant environ 2 heures à environ 60°C;

(7) à laver le substrat dans de l'eau distillée.